# FASCICULE DE BREVET EUROPEEN

(11) **EP 0 435 797 B1**
(45) Date de publication et mention de la délivrance du brevet: **12.10.1994**
(21) Numéro de dépôt: 90440117.1
(22) Date de dépôt: 10.12.1990
(51) Int. Cl.: A61K 9/06, A61K 33/00

(54) **Solution d'irrigation oculaire utilisée en thérapeutique pour la protection endothéliale et la conservation de la cornée**
Ophthalmologische therapeutische Lösung zur Schützung des Endotheliums und Konservierung der Hornhaut
Ophthalmic irrigation solution used in medicine for the endothelial protection and corneal preservation

(30) Priorité: 29.12.1989 FR 8917547
(43) Date de publication de la demande: 03.07.1991
(73) Titulaire: ANBEN, F-67000 Strasbourg (FR)
(72) Inventeur: Andermann, Guy, F-67000 Strasbourg (FR)

(56) Documents cités:
- WO-A-86/00228
- US-A- 4 620 979
- US-A- 4 837 021

## Description

Il a été démontré depuis un certain temps que la chirurgie oculaire du segment antérieur et postérieur de l'oeil affectait profondément l'intégrité de l'endothélium cornéen. Le traumatisme subi par les cellules endothéliales s'exprime par une augmentation de l'épaisseur cornéenne et une opacification plus ou moins importante de la cornée.

Au vue des dommages occasionnés, la recherche de solutions permettant de limiter la destruction cellulaire irréversible de la couche endothéliale, a été entreprise.

L'irrigation oculaire, nécessaire au cours de ces opérations chirurgicales du globe oculaire, a tour à tour progressé, en partant à l'origine de l'utilisation de solutions salines isotoniques stériles.

L'utilisation de telles solutions s'étant également révélée non dénuée de toxicité, on a préféré un moment utiliser la solution Lactate-Ringer, qui avait auparavant fait ses preuves pour l'irrigation d'autres tissus de l'organisme. Cependant, on s'est rapidement aperçu que les variations importantes de pH que pouvait subir cette solution, ne convenaient pas au maintien de l'intégrité cornéenne.

Au début des années 60, une solution, contenant les ions essentiels pour le maintien de la pompe endothéliale (Ca++, Mg++, K+, Na+) avait été commercialisée sous le nom B.S.S.. Cette composition constituait un progrès significatif, bien qu'elle se soit révélée par la suite, être à l'origine d'oedèmes cornéens du fait de sa **composition non-physiologique**.

En effet, seul une composition mimant parfaitement la composition de l'humeur aqueuse, est susceptible de causer un dommage minimum aux cellules de l'endothélium.

C'est pourquoi, une composition améliorée, contenant du glucose, du bicarbonate de sodium et du glutathion oxydé, a été décrite par la suite dans les brevets US 4.443.432 et 4.550.022.

Cette composition présente l'avantage d'être très proche de la composition de l'humeur aqueuse humaine, contenant, outre les ions essentielles décrits plus haut, un tampon physiologique (bicarbonate), un donneur d'énergie (glucose) et un "antioxydant", (le glutathion oxydé).

La supériorité de cette solution a été démontrée in vitro, en particulier sur le temps de récupération de la transparence cornéenne d'une part , et les jonctions intercellulaires d'autre part.

Cette préparation présente cependant **plusieurs désavantages**, en particulier dans son utilisation quotidienne. Il s'agit en effet d'une **préparation extemporanée**, se présentant en deux parties. Au moment de l'utilisation par le chirurgien, les constituants du flacon I, contenant du chlorure de calcium, du chlorure de magnésium et du glutathion oxydé, sont mis au contact avec les constituants du flacon II contenant les autres ions, le bicarbonate de sodium et le phosphate de sodium.

Cette **présentation malcommode** pour l'utilisateur, permet d'éviter une incompatibilité dans la composition qui conduirait à la précipitation de carbonate de calcium. En effet les solutions d'irrigation oculaire doivent
être limpides, sans suspensions ni particules. La présentation en 2 contenants permet ainsi une stabilité limitée à 6-8 heures après la préparation.

Il va sans dire que cette préparation est difficile à fabriquer, ce qui entraîne des coûts de production élevés et a pour conséquence **un prix final de produit élevé**. Son utilisation s'accompagne également d'autres désavantages non négligeables, comme **la possibilité de contamination microbienne** au cours de la préparation du mélange final. Des utilisateurs (chirurgiens et le personnel para-médical) peuvent également **oublier d'effectuer le mélange, ce qui serait préjudiciable au maintien de l'intégrité cornéenne** du fait d'un pH inadéquat de la solution I, non mélangée à la solution II.

D'autre part, **l'absence d'acide lactique dans la composition éloigne celle-ci de la définition de solution physiologique parfaite.** En effet, l'humeur aqueuse et le vitrée contiennent des ions lactates en quantité significative.

Il a maintenant été découvert qu'il était possible de réunir en une même solution tous les constituants (ou leurs analogues de structure) sans exception, des liquides baignant la cornée d'une part, et la rétine d'autre part.

En effet, la modification de la teneur de certains constituants comme les phosphates, les précautions au niveau de la fabrication du mélange, l'amélioration de certaines étapes du processus de fabrication, et le remplacement du glutathion oxydé par un analogue de structure, ont permis la réalisation industrielle d'une composition stable, efficace et économiquement avantageuse.

Ces modifications et améliorations concernent en particulier :
* la diminution de la concentration en phosphates ;
* la saturation de la solution en gaz carbonique, ce qui a pour effets d'éviter la précipitation de carbonate de calcium ;
* l'utilisation de contre ions organiques du calcium (comme l'acide glucoheptonique à titre d'exemple) au lieu de chlorure de calcium à des concentrations en glucoheptonate de calcium comprises entre 1-100 g/l (0,1-10 g %)
* le remplacement du glucose, fragile à la stérilisation par la chaleur, par d'autres fournisseurs d'énergie cellulaire, comme les diacides biologiques, tels l'acide fumarique, l'acide succinique, l'acide citrique et leurs sels pharmaceutiquement acceptables à des concentrations de 0,001 à 100 g/l (0,0001-10 g %), de structure comme dans la formule (avec n compris entre 1 et 3 : 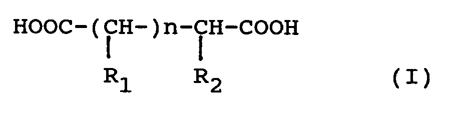 dans laquelle :
   R₁ est un atome d'hydrogène, un hydroxyle -OH,
   CH-R₁ peut être remplacé par un radical C=O
   R₂ est un atome d'hydrogène ou un hydroxyle -OH
   la liaison carbone-carbone est une simple liaison ou une double liaison
* le remplacement du glucose par des sucres complexes tels que l'acide gluconique, l'acide glucoheptonique, l'acide gluconoglucoheptonique , et leurs sels .
* l'association à des analogues de structure du glutathion réduit, comme l'acide gamma amino butyrique (GABA), à des concentrations comprises entre 10-10 000 mg/l (0,001-1 g %), ou ses analogues de structure gabaergiques, ainsi que ses sels pharmaceutiquement acceptables, comme dans la formule suivante (avec n compris entre 1 et 5 ): 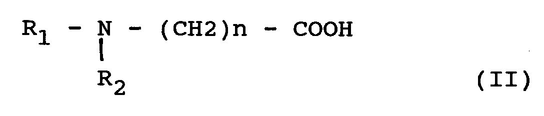 dans laquelle :
   R₁ est un atome d'hydrogène, un groupe alkyle inférieur, à chaine droite de 1 à 3 atomes de carbone, comprenant les radicaux méthyle, éthyle et propyle
   R₂ est un atome d'hydrogène,un groupe alkyle inférieur, à chaine droite de un à dix atomes de carbone, ou un radical -NH₂ comprenant les radicaux méthyle, éthyle et propyle,
   Parmi les composés typiques représentés par la formule (II) et utiles en tant que composés actifs des compositions d'irrigation oculaires conformes à la présente invention, on encore peut citer, , les composés suivants: l'acide aminocaproique, le glycocolle, la diméthylglycine, la sarcosine.
* l'association à des monoacides, comme l'acide lactique et ses sels pharmaceutiquement acceptables

Ces modifications conduisent à une solution parfaitement limpide, pouvant être stérilisée par la chaleur sèche ou humide. Ces opérations de stérilisation par la chaleur sèche ou humide, conduisent à une solution stérile et apyrogène, ce qui est une des caractéristiques essentielles d'une telle composition.

Outre ces composés utiles à l'activité de la composition, on doit obligatoirement leur associer des sels minéraux comme le chlorure de sodium, le chlorure de potassium, le chlorure de magnésium, le bicarbonate de sodium, en quantités suffisantes pour former une solution aqueuse isotonique et stable. Ces constituants ont pour rôle de créer un milieu physiologique favorable, qui mime la constitution de l'humeur aqueuse naturelle.

De plus, l'association nouvelle permet d'aboutir à une solution dont les valeurs extrêmes de pH ne varient pas dans des limites comprises au-delà de 6,8 et 7,6.
L'osmolarité de ces solutions est comprise entre 290 et 310 mOsm.

Toutes ces caractéristiques permettent d'assurer la formulation de compositions aussi proches que possible de l'humeur aqueuse physiologique.

Les résultats pré-cliniques et cliniques figurant ci-après montrent la supériorité du produit de l'invention sur les produits de comparaison, comme la solution BSS , ou la solution Lactate-Ringer.

Les composés décrits dans la présente invention ont été reconnus comme jouant un rôle de protection et de conservation de la cornée au cours d'interventions chirurgicales dans la sphère oculaire. Ces composés, associés à des donneurs d'énergie comme l'acide glucoheptonique ou l'acide lactique et des ions minéraux ont montré un effet de déturgescence tout à fait remarquable sur l'endothélium cornéen.

### Exemple de préparation : (Cet exemple ne décrit pas un mode de réalisation preferentiel de la présente invention)

| | |
|---|---|
| - Acide gamma amino butyrique | 1 mg/l à 100 mg/l |
| - Chlorure de potassium | 35 mg/l à 500 mg/l |
| - Glucoheptonate de calcium | 46 mg/l à 800 mg/l |
| - Chlorure de Magnésium (hexahydrate) | 15 mg/l à 450 mg/l |
| - Bicarbonate de sodium | 2 g/l à 3 g/l |
| - Phosphate acide de sodium (dihydrate) | 20 mg/l à 80 mg/l |
| - Lactate de sodium | 250 mg/l % à 1,5 g/l |
| - Chlorure de sodium | 5 g/l à 8 g/l |

chacune des quantités étant exprimées en poids/volume.

L'efficacité de la composition suivante a été étudiée du point de vue de son activité thérapeutique in vitro et in vivo :

### Essais in vitro sur la cornée de bovin

Des yeux de bovins ont été prélevés immédiatement après leur abattage, et sont acheminés dans les meilleurs délais au laboratoire d'essais où les cornées sont prélevées avant d'être prégonflées dans des cellules de perfusion en plexiglass. Au bout de 2 heures, les cornées ont ainsi acquis une épaisseur de 800 à 900 microns.

Les cellules de perfusion se composent de deux demi-cellules, maintenues l'une contre l'autre par une jaquette en aluminium thermostatable. Ce dispositif permet de fixer une cornée par l'intermédiaire d'une couronne de sclérotique de 5 mm.

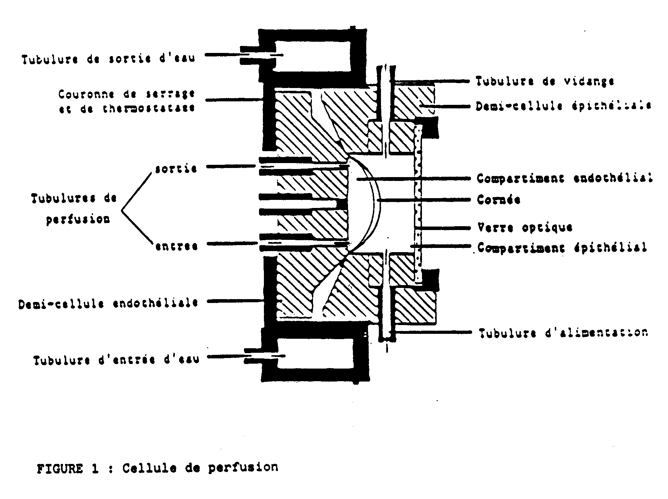

Le compartiment endothélial est perfusé en continu par l'intermédiaire d'une tubulure périphérique, qui amène les solutions testées au niveau de l'endothélium.
Une seconde tubulure diamétralement opposée, permet la sortie du perfusat. La figure 1 est un schéma général du dipositif.

Un bain thermostaté permet de maintenir une température de 37°C à l'intérieur de la cellule de perfusion. Les mesures répétées d'épaisseur cornéenne sont effectuées tout au long de l'expérience de perfusion, avec une lampe à fente de type Haag-Streit munie d'un pachymètre.

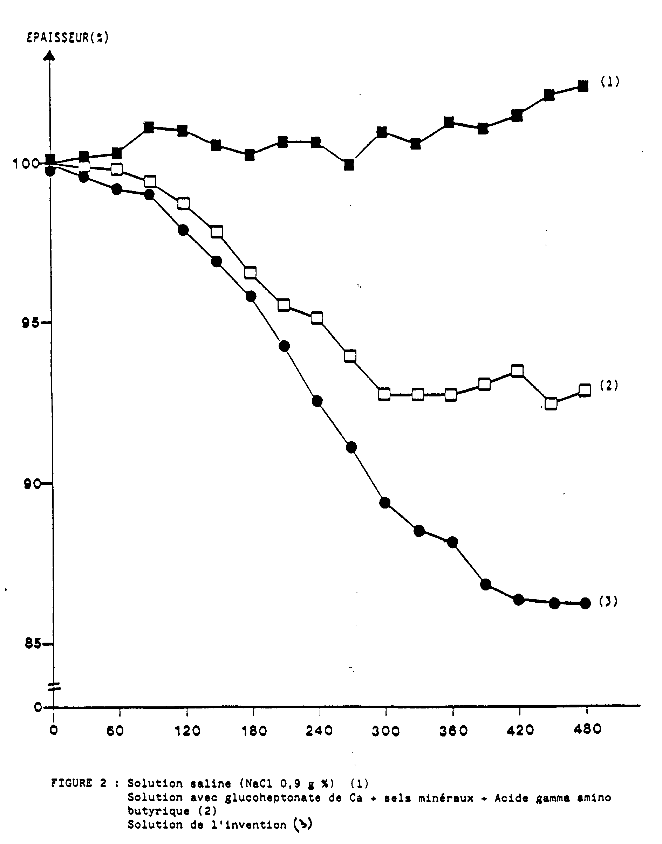

L'expérience compare la déturgescence cornéenne au cours de six heures de contact d'une solution saline d'une part, avec une solution contenant un donneur d'énergie (sel soluble d'acide glucoheptonique) d'autre part, et la solution test dont la composition figure ci-dessus. La figure 2 montrent que la solution à 0,9 g % de chlorure de sodium n'entraine aucune déturgescence de la cornée. Au contraire, celle-ci continue à gonfler, alors que la solution associant des sels minéraux, le sel de calcium de l'acide glucoheptonique, le sel sodique de l'acide lactique et l'acide gamma amino butyrique, permet une déturgescence cornéenne dès la deuxième heure. Le maintien de cette fonction de "pompe épithéliale" se poursuit jusqu'à la 6ème heure.
L'efficacité maxima est cependant obtenue avec la solution contenant un sel d'acide lactique, un sel calcique d'acide glucoheptonique, du bicarbonate de sodium et de l'acide gamma amino butyrique, suivant la composition décrite plus haut.

### Essais in vivo chez l'homme

Les essais cliniques entrepris sur un grand nombre de malades ont été réalisés en comparant l'efficacité de la solution selon l'exemple décrit ci-dessus avec une solution de référence (BSS). Les deux produits ont été utilisés au cours d'interventions chirurgicales de la sphère oculaire. Les essais ont été conduits selon le mode du double insu, sur 80 patients des deux sexes. Le protocole d'essai a utilisé le comptage cellulaire endothélial au microscope spéculaire, ainsi que la mesure de l'épaisseur cornéenne pour évaluer l'efficacité thérapeutique des produits testés. Ces 2 paramètres objectifs d'évaluation ont été appréciés avant et après l'intervention chirurgicale. L'analyse statistique des résultats a permis de conclure que la solution de l'invention a permis de réduire significativement le pourcentage de perte cellulaire au cours des interventions pratiquées. Les résultats ont été particulièrement significatifs dans les opérations de longue durée, où le produit testé, dont la composition figure plus haut, s'est montré supérieur au produit de référence (BSS).

## Revendications (Revendications pour l'(les) Etat(s) contractant(s) suivant(s): AT, BE, CH, DE, DK, GB, IT, LI, NL, SE)

1. Composition thérapeutique utilisée en tant que solution d'irrigation intraoculaire, caractérisée en ce qu'elle comprend :
a/ de 10 mg/l à 10 000 mg/l (0,001 - 1 g %) de dérivés gabaergiques choisis parmi les composés de formule générale : 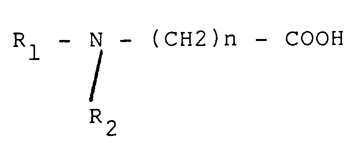 dans laquelle :
n est compris entre 1 et 5
R₁ est un atome d'hydrogène, un groupe alkyle inférieur, à chaîne droite de un à trois atomes de carbone
R₂ est un atome d'hydrogène, un groupe alkyle inférieur, a chaîne droite de un à dix atomes de carbone
b/ de 1 g/l à 100 g/l (0,1 - 10 g %) de glucoheptonate de calcium
c/ un monoacide organique, comme l'acide lactique ou un de ses sels pharmaceutiquement acceptables.

2. Composition thérapeutique selon la revendication 1, caractérisée en ce qu'elle contient en outre des sels minéraux comme le chlorure de sodium, le chlorure de potassium, le chlorure de magnésium, le bicarbonate de sodium et le phosphate de sodium, en quantités suffisantes pour former une solution aqueuse isotonique et stable.

3. Composition thérapeutique selon la revendication 1 et 2, caractérisée en ce que le dérivé gabaergique est constitué par l'acide aminocaproique, le glycocolle, la diméthylglycine, la sarcosine, ou l'acide gamma amino butyrique lui-même.

4. Composition thérapeutique selon l'une quelconque des revendications 1 à 3, caractérisée par le fait que la solution soit limpide, stable à la chaleur, stérile et apyrogène.

5. Composition thérapeutique selon l'une quelconque des revendications 1 à 4, caractérisée par le fait que son pH soit compris entre 6,8 et 7,6.

6. Composition thérapeutique selon l'une quelconque des revendications 1 à 5, caractérisée par le fait que l'osmolarité de la solution soit comprise entre 290 et 310 mOsm.

## Revendications (Revendications pour l'(les) Etat(s) contractant(s) suivant(s): ES, GR)

1. Procédé de préparation d'une composition thérapeutique utilisée en tant que solution d'irrigation intraoculaire, caractérisé en ce qu'il consiste à associer les différents constituants suivants :
a/ de 10 mg/l à 10 000 mg/l (0,001 - 1 g %) de dérivés gabaergiques choisis parmi les composés de formule générale : 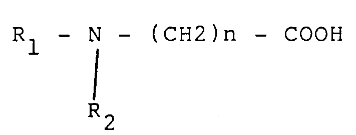 dans laquelle :
n est compris entre 1 et 5
R₁ est un atome d'hydrogène, un groupe alkyle inférieur, à chaîne droite de un à trois atomes de carbone
R₂ est un atome d'hydrogène, un groupe alkyle inférieur, à chaîne droite de un à dix atomes de carbone,
b/ de 1 g/l à 100 g/l (0,1 - 10 g %) de glucoheptonate de calcium,
c/ un monoacide organique, comme l'acide lactique ou un de ses sels pharmaceutiquement acceptables.

2. Procédé de préparation d'une composition thérapeutique selon la revendication 1, caractérisé en ce qu'il consiste à associer en outre des sels minéraux comme le chlorure de sodium, le chlorure de potassium, le chlorure de magnésium, le bicarbonate de sodium et le phosphate de sodium, en quantités suffisantes pour former une solution aqueuse isotonique et stable.

3. Procédé de préparation d'une composition thérapeutique selon la revendication 1 et 2, caractérisé en ce que le dérivé gabaergique est constitué par l'acide aminocaproique, le glycocolle, la diméthylglycine, la sarcosine, ou l'acide gamma amino butyrique lui-même.

4. Procédé de préparation d'une composition thérapeutique selon l'une quelconque des revendications 1 à 3, caractérisé par le fait que la solution soit limpide, stable à la chaleur, stérile et apyrogène.

5. Procédé de préparation d'une composition thérapeutique selon l'une quelconque des revendications 1 à 4, caractérisé par le fait que son pH soit compris entre 6,8 et 7,6.

6. Procédé de préparation d'une composition thérapeutique selon l'une quelconque des revendications 1 à 5, caractérisé par le fait que l'osmolarité de la solution soit comprise entre 290 et 310 mOsm.

## Claims (Claims for the following Contracting State(s): AT, BE, CH, DE, DK, GB, IT, LI, NL, SE)

1. A therapeutic composition used as an intraocular irrigating solution characterized by a combination of the following compounds:
a. from 10 mg/l to 10 000 mg/l (0.001-1 g%) of gabaergic derivatives having the following structure: 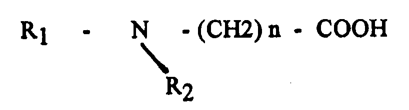 wherein
R1 is a hydrogen atom or a C1-C3 straight alkyl chain
R2 is a hydrogen atom or a C1-C10 straight alkyl chain
n is from 1 to 5
b. from 1g/l to 100 g/l (0.1-10 g%) of calcium glucoheptonate
c. an organic monoacid, such as lactic acid or one of its pharmaceutically acceptable salts

2. A therapeutic composition according to claim 1, characterized by a combination of mineral salts such as sodium chloride, potassium chloride, magnesium chloride,bicarbonate chloride and sodium phosphate in sufficicient quantity to constitute an isotonic and stable aqueous solution

3. A therapeutic composition according to claim 1 and 2 wherein the gabaergic derivatives are consisting of aminocaproic acid, glycine, dimethylglycine, sarcosine, or gamma amino butyric acid

4. A therapeutic composition according to at least one of claims 1 to 3, wherein the irrigating solution is clear, heat stable, sterile and non pyrogenic.

5. A therapeutic composition according to at least one of claims 1 to 4, characterized by a pH in the range of 6.8 to 7.2.

6. A therapeutic composition according to at least one of claims 1 to 5, characterized by an osmolality in the range of 290 mOsm and 310 mOsm.

## Claims (Claims for the following Contracting State(s): ES, GR)

1. A method for producing a therapeutic composition used as an intraocular irrigating solution characterized by a combination of the following compounds:
a. from 10 mg/l to 10 000 mg/l (0.001-1 g%) of gabaergic derivatives having the following structure: 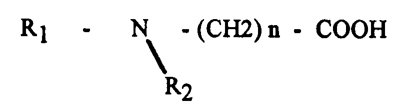 wherein
R1 is a hydrogen atom or a C1-C3 straight alkyl chain
R2 is a hydrogen atom or a C1-C10 straight alkyl chain
n is from 1 to 5
b. from 1g/l to 100 g/l (0.1-10 g%) of calcium glucoheptonate
c. an organic monoacid, such as lactic acid or one of its pharmaceutically acceptable salts

2. A method according to claim 1, characterized by a combination of mineral salts such as sodium chloride, potassium chloride, magnesium chloride,bicarbonate chloride and sodium phosphate in sufficicient quantity to constitute an isotonic and stable aqueous solution

3. A method according to claims 1 and 2 wherein the gabaergic derivatives are consisting of aminocaproic acid, glycine, dimethylglycine, sarcosine, or gamma amino butyric acid

4. A method according to at least one of claims 1 to 3, wherein the irrigating solution is clear, heat stable, sterile and non pyrogenic.

5. A method according to at least one of claims 1 to 4, characterized by a pH in the range of 6.8 to 7.2.

6. A method according to at least one of claims 1 to 5, characterized by an osmolality in the range of 290 mOsm and 310 mOsm.

## Patentansprüche (Patentansprüche für folgende(n) Vertragsstaat(en): AT, BE, CH, DE, DK, GB, IT, LI, NL, SE)

1. Eine therapeutische Mischung zum Benutzen als intraokulare Irrigationslösung, dadurch gekenntzeichnet, daß Sie folgende Bestandteile enthält:
a. 10mg/l bis 10 000 mg/l (0.001 g%-1 g%) von Gabaderivate folgender Struktur: 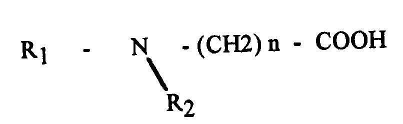 in der
R₁ ein Hydrogen oder eine gerade C₁-C₃ Alkylkette ist
R₂ ein Hydrogen oder eine gerade C₁-C₁₀ Alkylkette ist
n zwichen 1 und 5 liegt
b. 1g/l bis 100 g/l (0.1- 10 g %) Calcium Glucoheptonat
c. Eine organische Saüre, sowie Milchsaüre oder eines ihrer pharmazeutisch akzeptablen Natriumsalze

2. Eine therapeutische Mischung gemäß Anspruch 1, dadurch gekenntzeichnet, daß Sie eine Zusammensetzung von mineralischen Bestandteilen, wie zum Beispiel Natriumchlorid, Kaliumchlorid, Magnesiumchlorid, Natriumhydrogencarbonat, und Natriumhydrogenphosphat, in einer isotonischen stabilen Wasserlösung, enthält

3. Eine therapeutische Mischung gemäß Anspruch 1 und 2, dadurch gekenntzeichnet, daß es sich bei den Gabaderivate um Aminocaproicsaüre, glycin, dimethylglycin, sarcosin, und Gammaaminobutyricsaüre handelt

4. Eine therapeutische Mischung gemäß irgend einer der hiervor beschriebenen Ansprüche, dadurch gekenntzeichnet, daß es sich um eine klare, hitzstabile, sterile une nicht pyrogene Lösung handelt.

5. Eine therapeutische Mischung gemäß irgendeiner der hiervor beschriebenen Ansprüche, dadurch gekenntzeichnet, daß der pH-Wert der Lösung zwichen 6.8 und 7.6 liegt.

6. Eine therapeutische Mischung gemäß irgendeiner der hiervor beschriebenen Ansprüche, dadurch gekenntzeichnet, daß die Osmolalität der Lösung zwichen 290 mosm/kg und 310 mosm/kg liegt.

## Patentansprüche (Patentansprüche für folgende(n) Vertragsstaat(en): ES, GR)

1. Verfahren zur Herstellung einer therapeutischen Zubereitung zum Benutzen als intraokulare Irrigationslösung, dadurch gekenntzeichnet, daß Sie folgende Bestandteile enthält:
a. 10mg/l bis 10 000 mg/l (0.001 g%-1 g%) von Gabaderivate folgender Struktur: 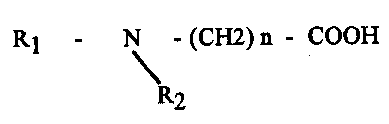 in der
R₁ ein Hydrogen oder eine gerade C₁-C₃ Alkylkette ist
R₂ ein Hydrogen oder eine gerade C₁-C₁₀ Alkylkette ist
n zwichen 1 und 5 liegt
b. 1g/l bis 100 g/l (0.1- 10 g %) Calcium Glucoheptonat
c. Eine organische Saüre, sowie Milchsaüre oder eines ihrer pharmazeutisch akzeptablen Natriumsalze

2. Verfahren zur Herstellung einer therapeutischen Zubereitung gemäß Anspruch 1, dadurch gekenntzeichnet, daß Sie eine Zusammensetzung von mineralischen Bestandteilen wie zum Beispiel Natriumchlorid, Kaliumchlorid, Magnesiumchlorid, Natriumhydrogencarbonat, und Natriumhydrogenphosphat, in einer isotonischen stabilen Wasserlösung enthält

3. Verfahren gemäß Anspruch 1 und 2, dadurch gekenntzeichnet, daß es sich bei den Gabaderivate um Aminocaproicsaüre, glycin, dimethylglycin, sarcosin, und Gamma-aminobutyricsaüre handelt

4. Verfahren gemäß irgend einer der hiervor beschriebenen Ansprüche, dadurch gekenntzeichnet, daß es sich um eine klare, Hitzstabile, sterile une nicht pyrogene Lösung handelt.

5. Verfahren gemäß irgendeiner der hiervor beschriebenen Ansprüche, dadurch gekenntzeichnet, daß der pH-Wert der Lösung zwichen 6.8 und 7.6 liegt.

6. Verfahren gemäß irgendeiner der hiervor beschriebenen Ansprüche, dadurch gekenntzeichnet, daß die Osmolalität der Lösung zwichen 290 mosm/kg und 310 mosm/kg liegt.
